# EUROPEAN PATENT SPECIFICATION

(11) **EP 2 571 557 B1**
(45) Date of publication and mention of the grant of the patent: **24.06.2015**
(21) Application number: 11721950.1
(22) Date of filing: 17.05.2011
(51) Int. Cl.: A61M 27/00

(54) **SYSTEMS FOR MEASURING REDUCED PRESSURE EMPLOYING AN ISOLATED FLUID PATH**
SYSTEME ZUM MESSEN DES REDUZIERTEN DRUCKS EINES ISOLIERTEN FLÜSSIGKEITSWEGS
SYSTÈMES POUR MESURER UNE PRESSION RÉDUITE EN UTILISANT UN CHEMIN FLUIDIQUE ISOLÉ

(30) Priority: 16.05.2011 US 201113108578; 18.05.2010 US 345821 P; 18.05.2010 US 345830 P; 17.11.2010 US 414738 P
(43) Date of publication of application: 27.03.2013
(73) Proprietor: KCI Licensing, Inc., San Antonio TX 78249-2248 (US)
(72) Inventor: LOCKE, Christopher, Brian, Bournemouth Dorset BH9 35D (GB); TOUT, Aidan, Marcus, Alderbury Wiltshire SP5 3FE (GB)
(74) Representative: Cordina, Kevin John
(86) International application number: PCT/US2011/036879
(87) International publication number: WO 2011/146533

(56) References cited:
- WO-A1-98/19145
- WO-A1-2007/087811
- DE-A1- 3 209 661
- GB-A- 2 342 584
- US-A1- 2003 097 100

## Description

### BACKGROUND

The present disclosure relates generally to reduced-pressure medical treatment systems and, more particularly, but not by way of limitation, to systems for measuring reduced pressure that employ an isolated fluid path.

Clinical studies and practice have shown that providing a reduced pressure in proximity to a tissue site augments and accelerates the growth of new tissue at the tissue site. The applications of this phenomenon are numerous, but application of reduced pressure has been particularly successful in treating wounds. This treatment (frequently referred to in the medical community as "negative pressure wound therapy," "reduced pressure therapy," or "vacuum therapy") provides a number of benefits, which may include faster healing and increased formulation of granulation tissue. Typically, reduced pressure is applied to tissue through a porous pad or other manifold device. The porous pad contains cells or pores that are capable of distributing reduced pressure to the tissue and channeling fluids that are drawn from the tissue. At times, it may be desirable to determine the reduced pressure involved at the tissue site. For example, it may be desirable to ascertain that the reduced pressure is in a therapeutic range.

WO 2007/087811 A1 describes a suction system for removal of exudate from a wound. A deflectable member and a pressure sensing device are provided.

US 2003/0097100 A1 describes a personally portable vacuum desiccator with a low pressure vacuum pump.

### SUMMARY

The invention is defined by independent claim 1.

According an illustrative, non-limiting embodiment, a system for treating a tissue site on a patient with reduced pressure includes a treatment manifold for deploying proximate to the tissue site, a sealing member for forming a fluid seal over the treatment manifold and a portion of the patient's epidermis, a reduced-pressure source for providing reduced pressure, and a reduced-pressure delivery conduit for fluidly coupling to the treatment manifold and to the reduced-pressure source. The reduced-pressure delivery conduit is for delivering treatment-reduced-pressure to the treatment manifold. The system further includes a reduced-pressure assessment conduit for fluidly coupling to the tissue site and an assessment chamber. The assessment chamber is for fluidly coupling to the reduced-pressure assessment conduit and for receiving an assessment-reduced-pressure from the tissue site. The assessment chamber includes a sealed enclosure having a first moveable portion on a wall. The first moveable portion is operable to move under the influence of reduced pressure. The system also includes a first pressure detector proximate to the first moveable portion of the assessment chamber. The first pressure detector is fluidly isolated from the assessment chamber and is operable to sense displacement of the first moveable portion.

According to an illustrative, non-limiting example, a method for treating a tissue site on a patient with reduced pressure includes disposing a treatment manifold proximate to the tissue site, disposing a sealing member over the treatment manifold and a portion of the patient's epidermis to form a fluid seal, providing a reduced-pressure source, fluidly coupling a reduced-pressure delivery conduit to the treatment manifold and to the reduced-pressure source, providing an assessment chamber, and fluidly coupling a reduced-pressure assessment conduit to the assessment chamber and to the tissue site for delivering an assessment-reduced-pressure to the assessment chamber. The assessment chamber has a first moveable portion on a wall. The method further includes disposing a first pressure detector proximate to the first moveable portion of the assessment chamber. The first pressure detector is fluidly isolated from the assessment chamber. The method also includes using the first pressure detector to sense displacement of the first moveable portion.

According to another illustrative, non-limiting example, a method for manufacturing a system for measuring reduced pressure at a tissue site on a patient includes forming an assessment chamber having a sealed enclosure with a first moveable portion, and forming a reduced-pressure assessment conduit having a distal end and a proximal end. The reduced-pressure assessment conduit is for fluidly coupling at the distal end to the tissue site and at the proximal end to the assessment chamber. The method also includes forming a first pressure detector disposed proximate to the first moveable portion of the assessment chamber and fluidly isolated from the assessment chamber. The first pressure detector is operable to sense displacement of the first moveable portion.

Other objects and advantages of the illustrative embodiments will become apparent with reference to the drawings and detailed description that follow.

### BRIEF DESCRIPTION OF THE DRAWINGS

FIGURE 1 is a schematic diagram with a portion shown in cross section of an illustrative, non-limiting embodiment of a reduced-pressure treatment system employing a subsystem for measuring reduced pressure that includes a first pressure detector isolated from an assessment chamber;
FIGURE 2 is a cross section of an illustrative, non-limiting embodiment of a combination conduit taken along line 2-2 in FIGURE 1;
FIGURE 3 is a schematic diagram of an illustrative, non-limiting embodiment of a reduced-pressure therapy unit;
FIGURE 4A is a schematic diagram of an illustrative, non-limiting embodiment of a pressure detector utilizing an electromagnetic coil and placed proximate to a first diaphragm having ferrite;
FIGURE 4B is the pressure detector of FIGURE 4A shown with the first diaphragm displaced;
FIGURE 5A is a schematic diagram of another illustrative, non-limiting embodiment of a pressure detector utilizing a Hall Effect sensor and placed proximate to a first diaphragm, which has a permanent magnet;
FIGURE 5B is the pressure detector of FIGURE 5A shown with the first diaphragm displaced;
FIGURE 6A is a schematic diagram of an illustrative, non-limiting embodiment of a pressure detector having a capacitive sensor proximate to a first diaphragm, which has ferrite;
FIGURE 6B is the pressure indicator of FIGURE 6A shown with the first diaphragm displaced;
FIGURE 7A is a schematic diagram of an illustrative, non-limiting embodiment of a pressure detector that includes an ultrasonic sensor and placed proximate to a first diaphragm;
FIGURE 7B is the pressure detector of FIGURE 7A shown with the first diaphragm displaced;
FIGURE 8A is a schematic diagram of an illustrative, non-limiting embodiment of a pressure detector including an infrared sensor and placed proximate to a first diaphragm, which has a reflector; and
FIGURE 8B is the pressure detector of FIGURE 8A shown with the first diaphragm displaced.

### DETAILED DESCRIPTION OF ILLUSTRATIVE EMBODIMENTS

In the following detailed description of the illustrative embodiments, reference is made to the accompanying drawings that form a part hereof. These embodiments are described in sufficient detail to enable those skilled in the art to practice the invention, and it is understood that other embodiments may be utilized and that logical structural, mechanical, electrical, and chemical changes may be made without departing from the scope of the invention. To avoid detail not necessary to enable those skilled in the art to practice the embodiments described herein, the description may omit certain information known to those skilled in the art. The following detailed description is not to be taken in a limiting sense, and the scope of the illustrative embodiments are defined only by the appended claims.

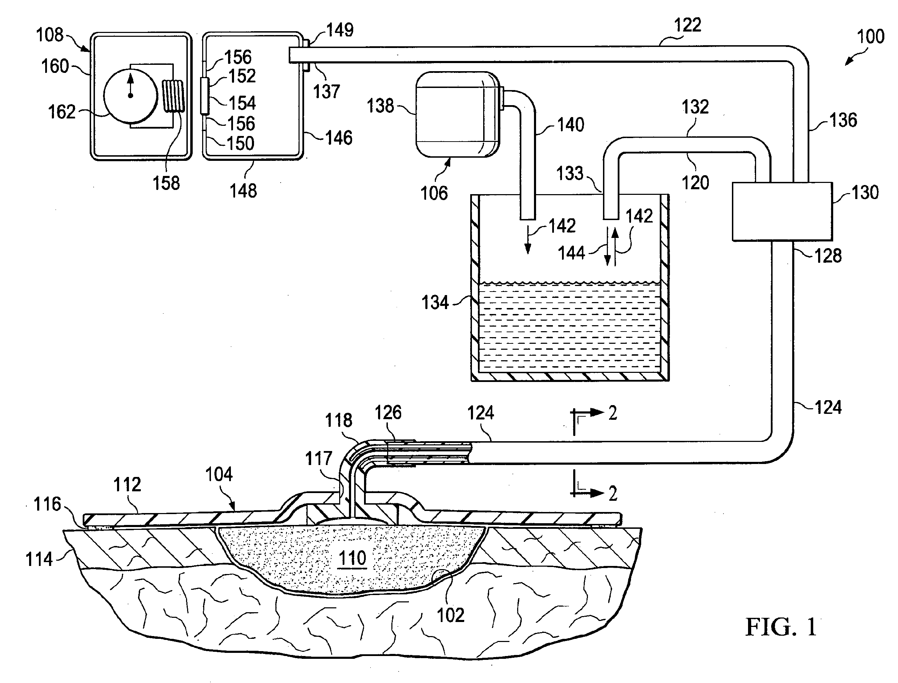

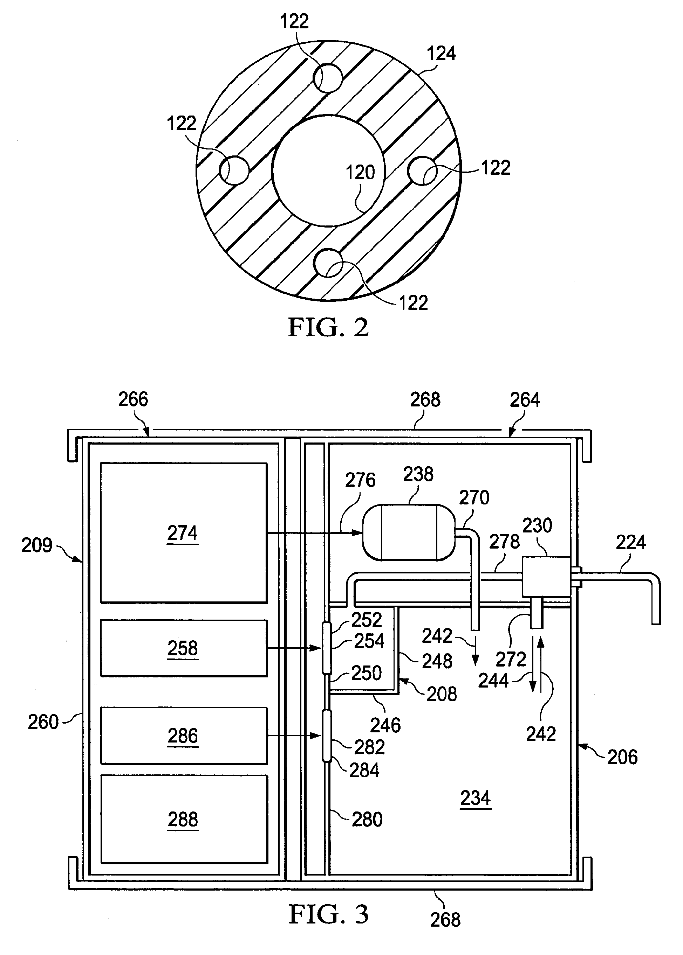

Referring to the drawings and primarily to FIGURES 1 and 2, a system 100 for treating a tissue site 102 on a patient with reduced pressure is presented. The system 100 includes a dressing 104, a reduced-pressure subsystem 106, and a reduced-pressure assessment subsystem 108. The reduced-pressure assessment subsystem 108 allows the reduced pressure at the tissue site 102 to be assessed with respect to pressure level while avoiding exposure of high-value components to contaminated fluids, which may be either liquids or gasses. As used herein, "or" does not require mutual exclusivity. The tissue site 102 may be the bodily tissue of any human, animal, or other organism, including bone tissue, adipose tissue, muscle tissue, dermal tissue, vascular tissue, connective tissue, cartilage, tendons, ligaments, or any other tissue. Fluid contaminants may include, without limitation, proteins, volatile organic compounds (VOC), fatty acids, amine such as putrescine and beutenoic acid, and other contaminates. The reduced-pressure assessment subsystem 108 will work with any orientation with respect to the gravity field because the pressure acts in all directions.

The dressing 104 includes a treatment manifold 110, which is placed proximate to the tissue site 102. A manifold is a substance or structure that is provided to assist in applying reduced pressure to, delivering fluids to, or removing fluids from a tissue site 102. The treatment manifold 110 typically includes a plurality of flow channels or pathways that distribute fluids provided to and removed from the tissue site 102 in an area near the treatment manifold 110. In one illustrative embodiment, the flow channels or pathways are interconnected to improve distribution of fluids provided or removed from the tissue site 102. The treatment manifold 110 may be a biocompatible material that is capable of being placed in contact with the tissue site 102 and distributing reduced pressure to the tissue site 102. Examples of treatment manifolds 110 may include, for example, without limitation, devices that have structural elements arranged to form flow channels, such as, for example, cellular foam, open-cell foam, porous tissue collections, liquids, gels, and foams that include, or cure to include, flow channels. The treatment manifold 110 may be porous and may be made from foam, gauze, felted mat, or any other material suited to a particular biological application.

In one illustrative, non-limiting embodiment, the treatment manifold 110 is a porous foam and includes a plurality of interconnected cells or pores that act as flow channels. The porous foam may be a polyurethane, open-cell, reticulated foam, such as GranuFoam® material manufactured by Kinetic Concepts, Incorporated of San Antonio, Texas. In some situations, the treatment manifold 110 may also be used to distribute fluids such as medications, antibacterials, growth factors, and various solutions to the tissue site 102. Other layers may be included in or on the treatment manifold 110, such as absorptive materials, wicking materials, hydrophobic materials, and hydrophilic materials.

The dressing 104 further includes a sealing member 112 that covers the treatment manifold 110 and a portion of the patient's epidermis 114. An attachment device 116 may be used to help form a fluid seal between the sealing member 112 and the patient's epidermis 114. A reduced-pressure interface 118 may extend through the sealing member 112 to provide fluid access to the treatment manifold 110. The fluid seal is adequate to maintain reduced pressure at a desired site given the particular reduced-pressure source or subsystem involved.

The sealing member 112 may be any material that provides a fluid seal. The sealing member 112 may be, for example, without limitation, an impermeable or semipermeable, elastomeric material. Examples of elastomers may include, but are not limited to, natural rubbers, polyisoprene, styrene butadiene rubber, chloroprene rubber, polybutadiene, nitrile rubber, butyl rubber, ethylene propylene rubber, ethylene propylene diene monomer, chlorosulfonated polyethylene, polysulfide rubber, polyurethane, EVA film, co-polyester, and silicones. Additional, specific examples of sealing members 112 include a silicone drape, 3M Tegaderm® drape, acrylic drape such as one available from Avery Dennison Corporation of Pasadena, California.

The attachment device 116 may be used to hold the sealing member 112 against the patient's epidermis 114 or another layer, such as a gasket or additional sealing member: The attachment device 116 may take numerous forms. For example, without limitation, the attachment device 116 may be a medically acceptable, pressure-sensitive adhesive that extends about a periphery of the sealing member 112 or a hydrocolloid material.

The reduced pressure developed by the reduced-pressure subsystem 106 is delivered through a reduced-pressure delivery conduit 120 to the reduced-pressure interface 118. In one illustrative embodiment, the reduced-pressure interface 118 is a T.R.A.C.^{®} Pad or Sensa T.RA.C.^{®}Pad available from KCI of San Antonio, Texas. The reduced-pressure interface 118 allows the reduced pressure to be delivered to the treatment manifold 110. The reduced-pressure interface 118 is also typically fluidly coupled to a reduced-pressure assessment conduit 122, which may be a plurality of reduced-pressure assessment conduits.

The reduced-pressure assessment conduit 122 allows the reduced pressure at the tissue site 102 to be communicated for measurement purposes. As shown clearly in FIGURE 2, the reduced-pressure delivery conduit 120 and the reduced-pressure assessment conduit 122 may be combined into a combination conduit 124 over some or all of their length. In the embodiment shown in FIGURES 1 and 2, the distal end 126 of the combination conduit 124 is fluidly coupled to the reduced-pressure interface 118 and receives reduced pressure from the tissue site 102. A proximal end 128 of the combination conduit 124 may be fluidly coupled to a connector 130. A portion 132 of the reduced-pressure delivery conduit 120 is fluidly coupled between the connector 130 and a fluid reservoir 134. A proximal end 133 of the reduced-pressure delivery conduit 120 is fluidly coupled to the fluid reservoir 134. A portion 136 of the reduced-pressure assessment conduit 122 is fluidly coupled between the connector 130 and the reduced-pressure assessment subsystem 108. A proximal end 137 of the reduced-pressure assessment conduit 122 is fluidly coupled to the assessment chamber 146 and may include a hydrophobic filter 149.

The reduced-pressure subsystem 106 delivers reduced pressure to the dressing 104. The reduced-pressure subsystem 106 includes a reduced-pressure source 138 that provides reduced pressure. The reduced-pressure source 138 is fluidly coupled to the fluid reservoir 134 by a second reduced-pressure delivery conduit 140 to deliver reduced pressure 142, or treatment-reduced-pressure 142, thereto. The reduced-pressure source 138 may be any device for supplying a reduced pressure, such as a vacuum pump, wall suction, or other source. While the amount and nature of reduced pressure applied to a tissue site will typically vary according to the application, the reduced pressure will typically be between -5 mm Hg (-667 Pa) and -500 mm Hg (-66.7 kPa) and more typically between -75 mm Hg (-9.9 kPa) and - 300 mm Hg (-39.9 kPa).

Reduced pressure is a pressure less than the ambient pressure at a tissue site that is being subjected to treatment. In most cases, this reduced pressure will be less than the atmospheric pressure at which the patient is located. Alternatively, the reduced pressure may be less than a hydrostatic pressure at the tissue site. Unless otherwise indicated, quantitative values of pressure stated herein are gauge pressures. The reduced pressure delivered may be constant or varied (patterned or random) and may be delivered continuously or intermittently. Although the terms "vacuum" and "negative pressure" may be used to describe the pressure applied to the tissue site, the actual pressure applied to the tissue site may be more than the pressure normally associated with a complete vacuum. Consistent with the use herein, an increase in reduced pressure or vacuum pressure typically refers to a relative reduction in absolute pressure.

The reduced-pressure subsystem 106 includes the reduced-pressure source 138 that delivers reduced pressure 142 to the fluid reservoir 134. The treatment-reduced-pressure 142 is delivered to the portion 132 of the reduced-pressure delivery conduit 120. The treatment-reduced-pressure 142 is then delivered via the reduced-pressure interface 118 to the treatment manifold 110.

The reduced-pressure interface 118 may receive fluids 144 from the tissue site 102 that are delivered by the reduced-pressure delivery conduit 120 to the fluid reservoir 134. The portion 136 of the reduced-pressure assessment conduit 122 delivers an assessment-reduced-pressure to the reduced-pressure assessment subsystem 108. The assessment-reduced-pressure is reduced pressure that is communicated from the reduced-pressure interface 118 or the tissue site 102 for the purpose of measuring. The reduced-pressure assessment subsystem 108 includes an assessment chamber 146 that receives the assessment-reduced-pressure from the reduced-pressure assessment conduit 122. The hydrophobic filter 149 may be placed at the inlet where the reduced-pressure assessment conduit 122 enters the assessment chamber 146. The hydrophobic filter 149 is to help prevent fluids from entering the assessment chamber 146.

The assessment chamber 146 includes a sealed enclosure 148. The sealed enclosure 148 includes a wall 150 having a first movable portion 152, such as first diaphragm 154. The first movable portion may experience a mixed phase, e.g. gas and liquids, without compromising accuracy. Typically, the first diaphragm 154 is a sheet of semi-flexible material anchored at the sheet's periphery 156 to the wall 150. The first diaphragm 154 is operable to move at least slightly from a neutral position to a displaced position within the assessment chamber 146 under the influence of reduced pressure. The sealed enclosure 148 may include a vent in some embodiments to allow gas from the tissue site to be vented to atmosphere.

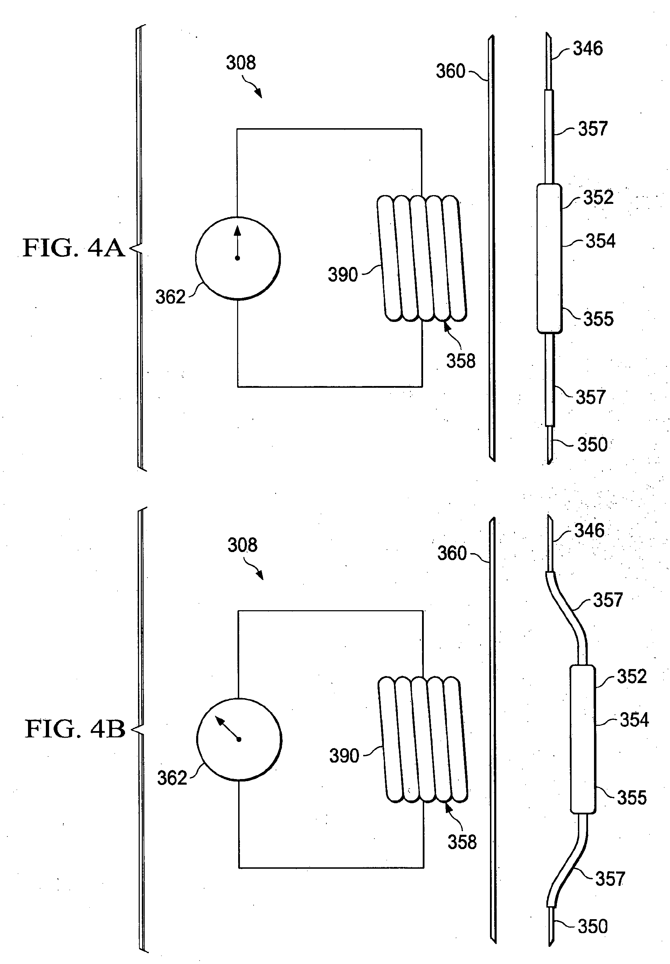

A first pressure detector 158 is located proximate to the first movable portion 152 and is operable to sense movement of the first movable portion 152. The first pressure detector 158 is fluidly isolated from the assessment chamber 146. Fluidly isolating the first pressure detector 158 from the assessment chamber 146 means that contaminates in any gas or liquid reaching the assessment chamber 146 will not reach or contaminate the first pressure detector 158. The first pressure detector 158 may be contained within an isolation chamber 160 or housing. As will be described in more detail below in connection with FIGURES 4A - 8B, the first pressure detector 158 may use a Hall Effect sensor, a capacitance sensor, ultrasonic sensor, infrared sensor, or other device to detect the movement of the first movable portion 152.

The reduced-pressure assessment subsystem 108 is operable to receive the assessment-reduced-pressure, which upon reaching a sufficient level will move the first movable portion 152 inward from a neutral position to a displaced position. The first pressure detector 158 senses the location of the first movable portion 152 and is able to provide an indication of a relative change in reduced pressure. The change in reduced pressure may be calibrated based on an initial measurement to reflect the reduced pressure experienced at the tissue site 102. The first pressure indicator 158 may present an indication of the relative change on indicator 162 or provide a signal for further processing or use.

In operation according to one illustrative embodiment, the treatment manifold 110 is placed proximate to the tissue site 102. The sealing member 112 is deployed using the attachment device 116. Thus, a fluid seal is formed between the sealing member 112 and a portion of the patient's epidermis 114. If not already installed, the reduced-pressure interface 118 may be applied through an aperture 117 in the sealing member 112.

If not already coupled, the reduced-pressure delivery conduit 120 and reduced-pressure assessment conduit 122 may be fluidly coupled to the reduced-pressure interface 118. Alternatively, as shown, a combination conduit 124 that includes the reduced-pressure delivery conduit 120 and the reduced-pressure assessment conduit 122 may be fluidly coupled to the reduced-pressure interface 118. The reduced-pressure delivery conduit 120 is fluidly coupled to the fluid reservoir 134. The reduced-pressure source 138 is fluidly coupled to the fluid reservoir 134 to provide reduced pressure to the fluid reservoir 134. Once activated, the reduced-pressure source 138 will deliver reduced pressure to the fluid reservoir 134 and to the tissue-site 102 via the reduced-pressure delivery conduit 120. Fluids will be typically moved into the reduced-pressure delivery conduit 120 and will flow to the fluid reservoir 134.

The reduced-pressure interface 118 allows the reduced pressure at one or more sampling sites to be communicated to one or more of the reduced-pressure assessment conduits 122. The reduced-pressure assessment conduit 122 delivers the assessment-reduced-pressure to the assessment chamber 146. As previously noted, the reduced pressure, upon reaching a sufficient level, moves the first movable portion 152 of the sealed enclosure 148 inward from a neutral position to a displaced position. The displacement of the first movable portion 152 is detected or sensed by the first pressure detector 158 and an indication of the reduced pressure may be made by the indicator 162 or a signal provided for further processing including display. The first pressure detector 158 may be coupled to provide a control signal to the reduced-pressure source 138 to provide feedback control in order to maintain a desired pressure or desired pressure range. The reduced-pressure assessment subsystem 108 may also include a user interface, e.g., a keypad and display, allowing the reduced pressure desired or the range desired to be entered or other control inputs to be received.

The system 100, and particularly the reduced-pressure assessment subsystem 108, allows the reduced pressure to be assessed with respect to pressure without contamination by gasses or liquids. The reduced-pressure assessment subsystem 108 may be made so that the assessment chamber 146 is made from relatively inexpensive components so as to conveniently allow the assessment chamber 146 to be disposed of after use while allowing the first pressure detector 158 to be used again without risking contamination. In this way, the reduced-pressure assessment subsystem 108 minimizes opportunities for the exposure of contaminated fluids to come into contact with the more high-valued items, or relatively expensive components.

Referring now primarily to FIGURE 3, another illustrative, non-limiting embodiment of a reduced-pressure subsystem 206 and a reduced-pressure assessment subsystem 208 are presented. FIGURE 3 is presented as a figurative cross section. In this illustrative embodiment, the reduced-pressure subsystem 206 and reduced-pressure assessment subsystem 208 are combined into a reduced-pressure therapy unit 209. The reduced-pressure therapy unit 209 may have a first portion 264, which may be designed for disposal and which isolates fluids in the first portion 264, and may have a second portion 266, which may be fluidly isolated from the first portion 264 and which may contain higher-valued items and components that may be reused. The first portion 264 and second portion 266 may be held proximate to one another either by an integral housing or by a bracket 268 or other device. In this illustrative embodiment, the reduced-pressure source 238 is a pump head that delivers reduced pressure 242 into a conduit 270 that delivers the reduced pressure into a fluid reservoir 234. The reduced pressure 242 is delivered into another conduit 272 that delivers the reduced pressure 242 to a connector 230. The connector 230 delivers the reduced pressure into a reduced-pressure delivery conduit (not explicitly shown) that is a part of a combination conduit 224. The combination conduit 224 delivers the reduced pressure to the tissue site. The second portion 266 of the reduced-pressure therapy unit 209 contains a pump control unit 274 that may provide pump energy to the pump head of the reduced pressure source 238 using a linking interface 276.

Assessment-reduced-pressure is delivered from the tissue site to the connector 230 using a reduced-pressure assessment conduit (not explicitly shown), which may be a part of the combination conduit 224. The assessment-reduced-pressure is delivered by a conduit 278 from the connector 230 to an assessment chamber 246 in a manner analogous to that shown in FIGURE 1. The assessment chamber 246 includes a sealed enclosure 248 that includes a wall 250. The wall 250 includes a first movable portion 252, such as a first diaphragm 254. A first pressure detector 258 may be included in the first portion 264 and may be aligned substantially with the first movable portion 252. The first pressure detector 258 is fluidly isolated from the assessment chamber 246. Under reduced pressure, the first movable portion 252 moves inward from a neutral position to a displaced position. The first pressure detector 258 is operable to sense displacement of the first movable portion 252 under the influence of reduced pressure and to indicate a change in reduced pressure on an indicator or with a signal. Thus, the first pressure detector 258 is operable to assess the pressure level at the tissue site.

The fluid reservoir 234 may include a wall 280 having a second movable portion 282, such as a second diaphragm 284. Under the influence of reduced pressure, the second movable portion 282 may move into the fluid reservoir 234 from a neutral position to a displaced position. A second pressure detector 286 may be included in the first portion 264 and may be substantially aligned with the second movable portion 282. The second pressure detector 286 is fluidly isolated from the fluid reservoir 234. The second pressure detector 286 is operable to sense displacement of the second movable portion 282 and to help determine the pressure or change in pressure within the fluid reservoir 234. Reduced-pressure data from the first pressure detector 258, which is indicative of the pressure at the tissue site, and data from the second pressure detector 286, which is indicative of the pressure in the fluid reservoir 234, may be used to assess performance of a reduced-pressure treatment system.

The pump control unit 274, the first pressure detector 258, the second detector 286 may be included in an isolation chamber 260 to help avoid dust and other small contaminates. The isolation chamber 260 may include one or more vents and may further include a power unit 288, such as a battery, that may provide electrical energy to the various components, e.g., the pump control unit 274, first pressure detector 258, and second pressure detector 286. In addition, the first portion 264 may include a user interface to receive inputs, such as a desired pressure. The first portion 264 may include an indicator (not shown) for visually indicating the pressure at the tissue or in the fluid reservoir 234. One should note that with the reduced-pressure therapy unit 209, the high-value components that may be desired for re-use are located within the first portion 264 and are fluidly isolated from the contaminated or potentially contaminated portions located in the second portion 266.

In the illustrative, non-limiting embodiments presented herein, numerous combinations of components may be used to sense the displacement of the movable portions 152, 252, 282. A number of illustrative, non-limiting embodiments for sensing the displacement will now be presented. Referring now primarily to FIGURES 4A and 4B, a portion of a reduced-pressure assessment subsystem 308 is presented. A portion of an assessment chamber 346 is shown with a wall 350 having a first movable portion 352, such as a first diaphragm 354. The first diaphragm 354 may include a target 355 and flexible or semi-flexible peripheral portion 357. In this illustrative, non-limiting embodiment, at least a portion of the first diaphragm 354, e.g. at least the target 355, is covered or made with a ferrite material.

Located proximate to the first movable portion 352, but fluidly isolated or separate from the interior of the assessment chamber 346, is a first pressure detector 358. The first pressure detector 358 may be located within an isolation chamber 360, which is partially shown. In this instance, the first pressure detector 358 includes an electromagnetic coil 390. Displacement of the first movable portion 352 with ferrite into the assessment chamber 346 as suggested in FIGURE 4B changes the inductance experienced by the electromagnetic coil 390. A change in the inductance may be measured and used to determine the displacement of the first movable portion 352. For example, as flux drops, one knows that the reduced pressure has increased, and the displacement of the first movable portion 352 may be calibrated to indicate the pressure inside the assessment chamber 346 relative to atmospheric pressure. The pressure change or pressure may be shown on an indicator 362 or a signal may be sent for further processing including displaying at another location.

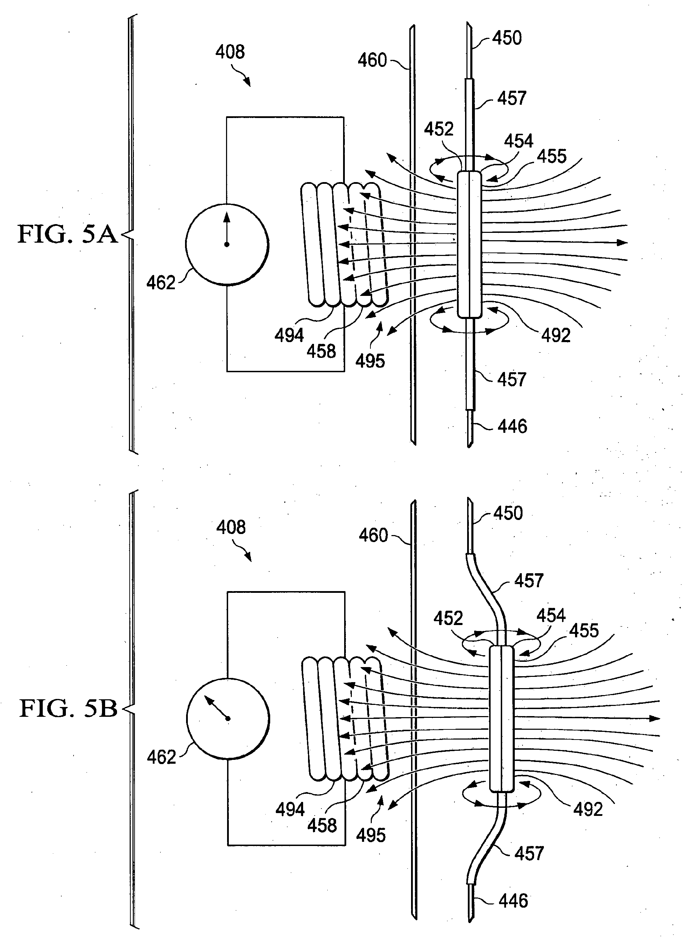

Referring now primarily to FIGURES 5A and 5B, another illustrative, non-limiting embodiment of a portion of a reduced-pressure assessment subsystem 408 is presented. A portion of an assessment chamber 446 is shown having a wall 450. The wall 450 includes a first movable portion 452, which may be a first diaphragm 454. The first diaphragm 454 includes a target 455 and a flexible or semi-flexible periphery 457. The target 455 includes a permanent magnet 492.

Located outside of the assessment chamber 446 and fluidly isolated from the assessment chamber 446 is a first pressure detector 458. The first pressure detector 458 may be within an isolation chamber 460, part of which is shown. The first pressure detector 458 may be a Hall Effect sensor 494 for sensing a magnetic field 495 or a change in the magnetic field 495. The change in the magnetic field 495 is caused by movement of the permanent magnet 492 on the first movable portion 452. The change in the magnetic field 495 may be used to produce a signal or indicate on indicator 462 the reduced pressure within the assessment chamber 446 or a change in reduced pressure.

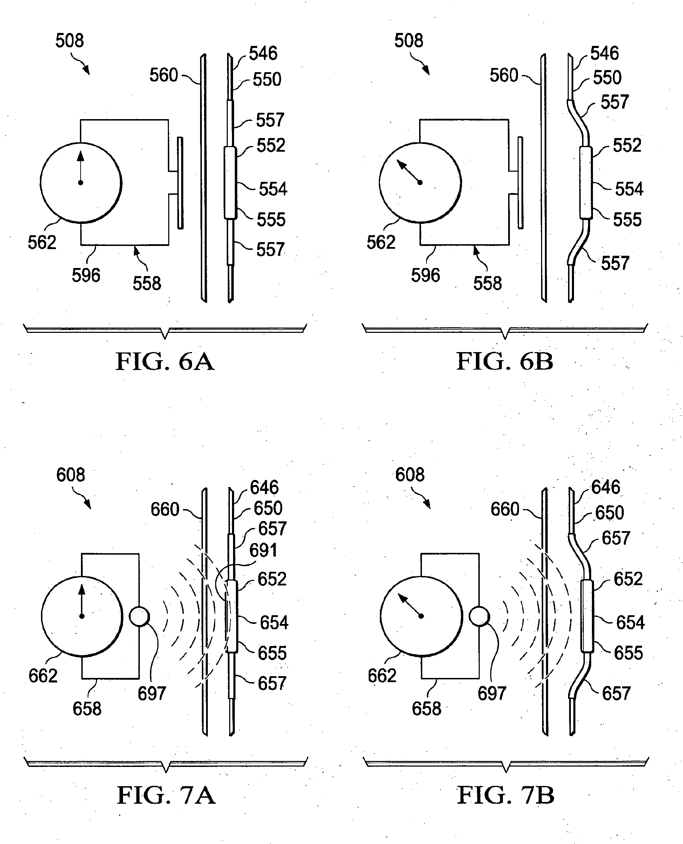

Referring now primarily to FIGURES 6A and 6B, another illustrative, non-limiting embodiment of a portion of a reduced-pressure assessment subsystem 508 is presented. A portion of an assessment chamber 546 is shown and includes wall 550. The wall 550 includes a first movable portion 552, such as a first diaphragm 554. The first diaphragm 554 includes a target 555 and a flexible or semi-flexible periphery 557. The target 555 on the first movable portion 552 includes ferrite or other material that may be sensed by a capacitive sensor 596.

A first pressure detector 558 may be located proximate to the first movable portion 552 and is fluidly isolated from the assessment chamber 546. The first pressure detector 558 may be within an isolation chamber 560, or housing. In this illustrative embodiment, the first pressure detector 558 is the capacitive sensor 596, and thus, displacement of the first movable portion 552 causes a change in capacitance that is sensed by the capacitance sensor 596. The change may be used to detect displacement of the first movable portion 552 such as is shown in FIGURE 6B. The displacement may be indicative of the pressure change or the pressure experienced within the assessment chamber 546 and may produce a signal or be shown on an indicator 562. In general, the capacitance between the first movable portion 552 and the capacitive sensor 596 is proportional to the square of the distance between them.

Referring now primarily to FIGURES 7A and 7B, a portion of an illustrative, non-limiting embodiment of a reduced-pressure assessment subsystem 608 is presented. The reduced-pressure assessment subsystem 608 includes assessment chamber 646, a portion of which is shown, having a wall 650. The wall 650 includes a first movable portion 652. The first movable portion 652 may be a first diaphragm 654. The first diaphragm 654 may include a target 655 having a flexible or semi-flexible portion 657 coupling the target 655 and the wall 650. The flexible or semi-flexible portion 657 allows movement of the target 655. Under reduced pressure, the first movable portion 652 moves inward into the assessment chamber 646 from a neutral position to a displaced position as shown in FIGURE 7B.

A first pressure detector 658 is fluidly separated from the assessment chamber 646 and is substantially aligned with the first movable portion 652. The first pressure detector 658 may be inside of an isolation chamber 660. In this embodiment, the first pressure detector 658 is an ultrasonic sensor 697 that sends out ultrasonic sound waves that are bounced off of a reflector 691 on the first movable portion 652. The ultrasonic sensor 697 senses displacement of the first movable portion 652 and develops a signal indicative of the displacement. The signal may be used to show a pressure measurement on an indicator 662 or for further processing.

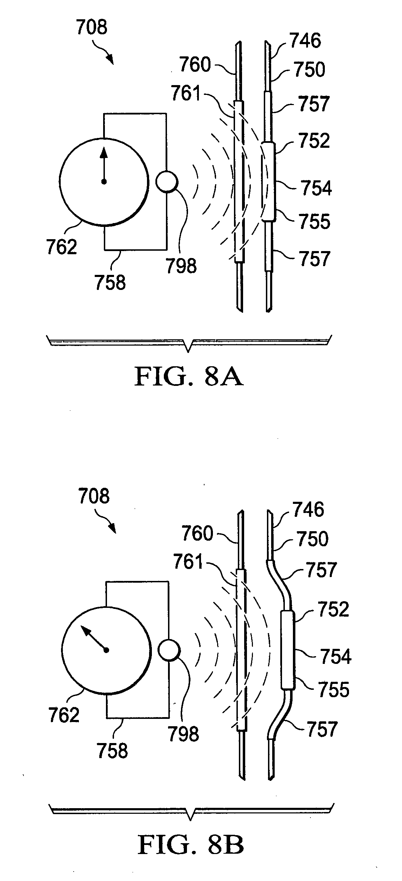

Referring now primarily to FIGURES 8A and 8B, another illustrative, non-limiting embodiment of a portion of a reduced-pressure assessment subsystem 708 is presented. The reduced-pressure assessment subsystem 708 includes an assessment chamber 746, which is partially shown. The assessment chamber 746 includes a wall 750 having a first movable portion 752. The first movable portion 752 may be a first diaphragm 754. The first diaphragm 754 may include a target 755 and a flexible or semi-flexible portion 757. The first movable portion 752 moves under the influence of reduced pressure and may move into the assessment chamber 746 from a neutral position to a displaced position.

A first pressure detector 758 may be located proximate to the first movable portion 752 and is fluidly isolated from the inside of the assessment chamber 746. The first pressure detector 758 may be contained within an isolation chamber 760. In this illustrative, non-limiting embodiment, the isolation chamber 760 includes a window 761. The window 761 allows at least infrared signals to travel through. The first pressure detector 758 includes the infrared sensor 798 that is operable to propagate an infrared wave through the window 761 to impact the first movable portion 752 and to determine the relative location of the first movable portion 752. The first movable portion 752 reflects the infrared wave. The first movable portion 752 moves into the assessment chamber 746 under the influence of reduced pressure as shown in FIGURE 8B. The infrared sensor 798 detects the displacement and is able to provide a signal or indicate, such as on indicator 762, the amount of displacement or the corresponding pressure.

Although the present invention and some of its advantages have been disclosed in the context of certain illustrative, non-limiting embodiments, it should be understood that various changes, substitutions, permutations, and alterations can be made without departing from the scope of the invention as defined by the appended claims. As one illustrative, non-limiting example, it should be noted that any of the portions of the reduced-pressure assessment subsystems presented in FIGURES 4A to 8B, may also be used with a second movable portion, such as second movable portion 282 in FIGURE 3.

It will be understood that the benefits and advantages described above may relate to one embodiment or may relate to several embodiments. It will further be understood that reference to "an" item refers to one or more of those items.

The steps of the methods described herein may be carried out in any suitable order, or simultaneously where appropriate.

Where appropriate, aspects of any of the embodiments described above may be combined with aspects of any of the other embodiments described to form further examples having comparable or different properties and addressing the same or different problems.

It will be understood that the above description of preferred embodiments is given by way of example only and that various modifications may be made by those skilled in the art. The above specification, examples and data provide a complete description of the structure and use of exemplary embodiments of the invention. Although various embodiments of the invention have been described above with a certain degree of particularity, or with reference to one or more individual embodiments, those skilled in the art could make numerous alterations to the disclosed embodiments without departing from the scope of the claims.

## Claims

1. A system for treating a tissue site on a patient with reduced pressure, the system comprising:
a treatment manifold (110) for deploying proximate to the tissue site (102);
a sealing member (112) for forming a fluid seal over the treatment manifold and a portion of the patient's epidermis;
a reduced-pressure source (138) for providing reduced pressure;
a reduced-pressure delivery conduit (120, 140) for fluidly coupling to the treatment manifold (110) and to the reduced-pressure source (138), the reduced-pressure delivery conduit (120, 140) for delivering treatment-reduced-pressure to the treatment manifold (110);
a reduced-pressure assessment conduit (122) for fluidly coupling to the tissue site (102);
an assessment chamber (148) for fluidly coupling to the reduced-pressure assessment conduit (122) for receiving an assessment-reduced-pressure from the tissue site (102), wherein the assessment chamber (148) comprises a sealed enclosure having a first moveable portion (152) on a wall and wherein the first moveable portion (152) is operable to move under the influence of reduced pressure; and
a first pressure detector (158) proximate to the first moveable portion (152) of the assessment chamber (148) and fluidly isolated from the assessment chamber (148), the first pressure detector (158) operable to sense displacement of the first moveable portion (152);
a fluid reservoir (134) for receiving fluids from the tissue site (102), wherein the fluid reservoir (134) is fluidly coupled to the reduced-pressure source (138) and the reduced-pressure delivery conduit (120),
**characterised in that** the fluid reservoir has a second moveable portion (282) on a wall; and
a second pressure detector (286) proximate to the second moveable portion (282) of the fluid reservoir and fluidly isolated from the fluid reservoir, the second pressure detector (286) operable to sense displacement of the second moveable portion (282).

2. The system of claim 1, further comprising an indicator (162) and wherein the first pressure detector (158) is operable to indicate on the indicator a change in relative reduced pressure based on sensing displacement of the first moveable portion (158).

3. The system of claim 1, wherein the first moveable portion (158) comprises a first diaphragm member with ferrite and the first pressure detector comprises an electromagnet coil for sensing changes in flux density.

4. The system of claim 1, wherein the first moveable portion (158) comprises a first diaphragm member with a permanent magnet coupled to the first diaphragm and the first pressure detector comprises an a Hall Effect sensor for sensing changes in location of the first diaphragm.

5. The system of claim 1, wherein the first moveable portion (158) comprises a first diaphragm member with a ferrite coupled to the first diaphragm and the first pressure detector comprises a capacitive sensor for sensing changes in location of the first diaphragm.

6. The system of claim 1, wherein the first moveable portion (158) comprises a first diaphragm member and the first pressure detector comprises an ultrasonic sensor for sensing , changes in location of the first diaphragm.

7. The system of claim 1, wherein the first moveable portion (158) comprises a first diaphragm member having a reflector, wherein the first detector further comprises a window on an exterior wall proximate the first diaphragm, and the first pressure detector comprises an infrared sensor for sensing changes in location of the first diaphragm.

## Patentansprüche

1. Ein System zum Behandeln einer Gewebestelle bei einem Patienten mit reduziertem Druck, das System aufweisend:
einen Behandlungsverteiler (110) zum Einsatz nahe der Gewebestelle (102);
ein Abdichtungsglied (112) zum Formen einer Flüssigkeitsabdichtung über dem Behandlungsverteiler und einem Teil von der Epidermis des Patienten;
eine Quelle (138) von reduziertem Druck zum Bereitstellen eines reduzierten Drucks;
eine Zustellleitung (120, 140) von reduziertem Druck zum Flüssigkeitsverbinden mit dem Behandlungsverteiler (110) und mit der Quelle (138) von reduziertem Druck, wobei die Zustellleitung (120, 140) von reduziertem Druck reduzierten Behandlungsdruck an den Behandlungsverteiler (110) zustellt;
eine Erfassungsleitung (122) von reduziertem Druck zum Flüssigkeitsverbinden mit der Gewebestelle (102);
eine Erfassungskammer (148) zum Flüssigkeitsverbinden mit der Erfassungsleitung (122) von reduziertem Druck zum Empfangen eines reduzierten Erfassungsdrucks von der Gewebestelle (102), wobei die Erfassungskammer (148) ein abgedichtetes Gehäuse mit einem ersten beweglichen Teil (152) an einer Wand aufweist und wobei das erste bewegliche Teil (152) betriebsfähig ist, um sich unter dem Einfluss von reduziertem Druck zu bewegen; und
einen ersten Druckdetektor (158) nahe dem ersten beweglichen Teil (152) von der Erfassungskammer (148) und flüssigkeitsisoliert von der Erfassungskammer (148), der erste Druckdetektor (158) ist betriebsfähig, um eine Verschiebung von dem ersten beweglichen Teil (152) wahrzunehmen;
einen Flüssigkeitsbehälter (134) zum Empfangen von Flüssigkeiten von der Gewebestelle (102), wobei der Flüssigkeitsbehälter (134) mit der Quelle (138) von reduziertem Druck und der Zustellleitung (120) von reduziertem Druck flüssigkeitsverbunden ist,
**dadurch gekennzeichnet, dass** der Flüssigkeitsbehälter einen zweiten beweglichen Teil (282) an einer Wand hat; und
einen zweiten Druckdetektor (286) nahe dem zweiten beweglichen Teil (282) von dem Flüssigkeitsbehälter und flüssigkeitsisoliert von dem Flüssigkeitsbehälter, der zweite Druckdetektor (286) ist betriebsfähig, um eine Verschiebung von dem zweiten beweglichen Teil (282) wahrzunehmen.

2. Das System von Anspruch 1, weiter aufweisend einen Anzeiger (162) und wobei der erste Druckdetektor (158) betriebsfähig ist, um über den Anzeiger eine Änderung in relativ reduziertem Druck basierend auf der Wahrnehmung der Verschiebung von dem ersten beweglichen Teil (158) anzuzeigen.

3. Das System von Anspruch 1, wobei der erste bewegliche Teil (158) einen ersten Membranteil mit Ferrit aufweist und der erste Druckdetektor eine Elektromagnetspule zum Wahrnehmen von Änderungen in einer Flussdichte aufweist.

4. Das System von Anspruch 1, wobei der erste bewegliche Teil (158) einen ersten Membranteil mit einem mit dem ersten Membran verbundenen Dauermagneten aufweist und der erste Druckdetektor einen Hall-Effekt-Sensor zum Wahrnehmen von Änderungen in einer Position von dem ersten Membran aufweist.

5. Das System von Anspruch 1, wobei der erste bewegliche Teil (158) einen ersten Membranteil mit einem mit dem ersten Membran verbundenen Ferrit aufweist und der erste Druckdetektor einen kapazitiven Sensor zum Wahrnehmen von Änderungen in einer Position von der ersten Membran aufweist.

6. Das System von Anspruch 1, wobei der erste bewegliche Teil (158) einen ersten Membranteil aufweist und der erste Druckdetektor einen Ultraschallsensor zum Wahrnehmen von Änderungen in einer Position von der ersten Membran aufweist.

7. Das System von Anspruch 1, wobei der erste bewegliche Teil (158) einen ersten Membranteil mit einem Reflektor aufweist, wobei der erste Detektor weiter ein Fenster auf einer Außenwand nahe der ersten Membran aufweist und der erste Druckdetektor einen Infrarotsensor zum Wahrnehmen von Änderungen in einer Position von der ersten Membran aufweist.

## Revendications

1. Système de traitement d'un site tissulaire sur un patient avec une pression réduite, le système comprenant :
un collecteur de traitement (110) à déployer à proximité du site tissulaire (102) ;
un élément d'étanchéité (112) pour former un joint étanche aux fluides sur le collecteur de traitement et une partie de l'épiderme du patient ;
une source de pression réduite (138) pour délivrer une pression réduite ;
un conduit de délivrance de pression réduite (120, 140) pour établir un couplage fluidique avec le collecteur de traitement (110) et avec la source de pression réduite (138), le conduit de délivrance de pression réduite (120, 140) visant à délivrer une pression réduite de traitement au collecteur de traitement (110) ;
un conduit d'évaluation de pression réduite (122) pour établir un couplage fluidique avec le site tissulaire (102) ;
une chambre d'évaluation (148) pour établir un couplage fluidique avec le conduit d'évaluation de pression réduite (122) afin de recevoir une pression réduite d'évaluation du site tissulaire (102), dans lequel la chambre d'évaluation (148) comprend une enceinte scellée ayant une première partie mobile (152) sur une paroi et dans lequel la première partie mobile (152) est à même de se déplacer sous l'influence de la pression réduite ; et
un premier détecteur de pression (158) à proximité de la première partie mobile (152) de la chambre d'évaluation (148) et isolé au plan fluidique de la chambre d'évaluation (148), le premier détecteur de pression (158) étant à même de détecter le déplacement de la première partie mobile (152) ;
un réservoir de fluide (134) pour recevoir des fluides du site tissulaire (102), dans lequel le réservoir de fluide (134) présente un couplage fluidique avec la source de pression réduite (138) et le conduit de délivrance de pression réduite (120), **caractérisé en ce que** le réservoir de fluide a une seconde partie mobile (282) sur une paroi ; et
un second détecteur de pression (286) à proximité de la seconde partie mobile (282) du réservoir de fluide et isolé au plan fluidique du réservoir de fluide, le second détecteur de pression (286) étant à même de détecter le déplacement de la seconde partie mobile (282).

2. Système selon la revendication 1, comprenant en outre un indicateur (162) et dans lequel le premier détecteur de pression (158) est à même d'indiquer sur l'indicateur un changement de la pression réduite relative sur la base de la détection du déplacement de la première partie mobile (158).

3. Système selon la revendication 1, dans lequel la première partie mobile (158) comprend un premier élément de diaphragme avec de la ferrite et le premier détecteur de pression comprend une bobine électromagnétique pour détecter les changements de densité du flux.

4. Système selon la revendication 1, dans lequel la première partie mobile (158) comprend un premier élément de diaphragme avec un aimant permanent couplé au premier diaphragme et le premier détecteur de pression comprend un capteur à effet Hall pour détecter les changements d'emplacement du premier diaphragme.

5. Système selon la revendication 1, dans lequel la première partie mobile (158) comprend un premier élément de diaphragme avec de la ferrite couplé au premier diaphragme et le premier détecteur de pression comprend un capteur capacitif pour détecter les changements d'emplacement du premier diaphragme.

6. Système selon la revendication 1, dans lequel la première partie mobile (158) comprend un premier élément de diaphragme et le premier détecteur de pression comprend un capteur ultrasonique pour détecter les changements d'emplacement du premier diaphragme.

7. Système selon la revendication 1, dans lequel la première partie mobile (158) comprend un premier élément de diaphragme ayant un réflecteur, dans lequel le premier détecteur comprend en outre une fenêtre sur une paroi externe à proximité du premier diaphragme et le premier détecteur de pression comprend un capteur infrarouge pour détecter les changements d'emplacement du premier diaphragme.
